# EUROPEAN PATENT APPLICATION

(11) **EP 4 464 247 A1**
(43) Date of publication of application: **20.11.2024**
(21) Application number: 23173552.3
(22) Date of filing: 16.05.2023
(51) Int. Cl.: A61B 5/1495, G01N 27/416, G01N 33/00

(54) **CALIBRATION AND/OR VERIFICATION TOOL**

(71) Applicant: SenTec AG, 4106 Therwil (CH)
(72) Inventor: SCHUMACHER, Peter Matthias, 3038 Kirchlindach (CH); FRAGKOS, Christos, Athens (GR); DE LANGE, Victoria Emily, 4600 Olten (CH)
(74) Representative: Hepp Wenger Ryffel AG

(57) **Abstract**

The invention is directed to a calibration and/or verification tool, an assembly and a method for calibrating and/or verifying systems for measuring gas concentrations of gases that have diffused transcutaneously. The calibration and/or verification tool (1) is a tool for calibrating and/or verifying a system (100) for measuring gas concentrations, with a sensor (101) for detection of transcutaneous gas, comprising a contact face (102) which is directable towards a measuring site on the skin of a human or animal subject. The calibration and/or verification tool (1) comprises a compartment (2) for encompassing a reference environment (3), an interface (4) for connecting the sensor (101), such that the contact face (102) is exposed to the reference environment (3) in the compartment and means for providing a well-defined gas concentration in the compartment. The calibration and/or verification tool (1) is a mobile stand-alone device and preferably a single-use and/or a disposable device.

## Description

The invention is directed to a calibration and/or verification tool, an assembly and a method for calibrating and/or verifying systems for measuring gas concentrations of gases that have diffused transcutaneously.

Current systems for measuring gas employ a variety of measurement principles. In medical technology, predominantly electrochemical sensors are used for measurement of gases that have diffused transcutaneously, i.e., that have diffused through the skin of a human patient or of an animal. Current sensors for measurement of transcutaneous CO2 are very sensitive and show good response times. Electrochemical sensors for measurement of transcutaneous gases are known for example from WO 2008/132205 and EP3024390B21.

Nevertheless, those sensors drift over longer measurement periods and require frequent calibrations. Therefore, at the point of use, users need to use rather bulky and expensive calibration instruments including a calibration gas supply. Sensor calibration and maintenance impair the usability of the technology.

Known calibration stations are integrated inside the measuring device. For example, EP0074498 B1 discloses a measuring system comprising a sensor being connected to a measuring device and a calibration system which combines the calibrator device and the measuring device.

In currently known calibration systems the user may insert a canister of test gas and may place the sensor into a docking station. Closing the docking station door may create a seal around the PCO2 sensing element by pressing the sensor against an O-ring. Software-controlled mechanical valves can then regulate the flow of gas from the canister to the sensor. This system suffers from several usability issues, which can create gas leaks and may lead to unsuccessful calibrations. Troubleshooting is time-consuming and can be frustrating for customers.

It is an object of the present invention to avoid the drawbacks of the state of the art and, in particular, to provide a simple, inexpensive, robust and precise tool and a method for calibrating and verifying of systems for measuring gas concentrations of gases that have diffused transcutaneously.

According to the invention this object is accomplished by a calibration and/or verification tool, an assembly and a method according to the independent patent claims.

The calibration and/or verification tool is a tool for calibrating and/or verifying a system for measuring gas concentrations. Said system comprises a sensor for detection of transcutaneous gas and a contact face which is directable towards a measuring site on the skin of a human or animal subject.

The calibration and/or verification tool comprises a compartment for encompassing a reference environment. The calibration and/or verification tool also comprises an interface for connecting the sensor, such that the contact face is exposed to the reference environment in the compartment. The calibration and/or verification tool further comprises means for providing a well-defined gas concentration in the compartment.

The compartment may have a volume of 0.05ml to 15ml.

The interface may comprise a receptacle for a corresponding part of the sensor. The interface may comprise fastening elements, like clips, clamping elements, threads, a locking ring with castellated slots or a closure plug with castellated lugs for a bayonet closure.

As sensors of systems for measuring gas concentrations drift over time, it is necessary to check if the setting is still acceptable (verifying) and/or to recalibrate the system (calibrating). In any case, a reference environment may be presented to the system.

After connecting the sensor to the calibration and/or verification tool the contact face is exposed to a volume containing a reference environment. The reference environment may be provided by a reference gas in a static or in a dynamic way.

When the calibration and/or verification tool has been connected to the system for measuring gas concentrations, the system should provide a value for the gas concentration which corresponds to the respective gas concentration in the compartment. Otherwise, the system has to be adjusted until a respective value is provided. If the value provided by the system does not correspond to the pre-set gas concentration in the compartment, the user understands that the system is not suitable for measurements unless it is recalibrated.

Preferably, the calibration and/or verification tool comprises a detection means, which may be recognized and/or read out by the system for measuring gas concentrations. The detection means may be formed by an RFID and/or an NFC tag, magnetic switch or a hall sensor. The detection means may comprise information about the calibration and/or verification tool, such as a serial number, an expiration date and so on.

The detection means may provide information about whether the calibration and/or verification tool is connected to the system for measuring gas concentrations. When using such means, the calibration process can be fully automated, requiring minimal user interaction.

Within this context a "well-defined" gas concentration in the compartment means that a concentration is provided with a precision range which corresponds to the measurement accuracy of the system.

The calibration and/or verification tool is a mobile, stand-alone device and preferably a single-use and/or a disposable device.

Within this application "stand-alone" means separate, in particular from the system, and the device preferably does not comprise electronic components, a display and an electronic interface besides passive detection means.

However, a display may be needed during use. This display may be provided during use by the system.

Within this application "mobile" means, that the calibration and/or verification tool can be moved, in particular away from the system. The calibration and/or verification tool may for example be used with different systems for which the interface is appropriate and/or with any system for which the interface is suitable and which needs verification and/or calibration.

The calibration and/or verification tool may be a handheld device.

The calibration and/or verification tool may be small and may have a total volume of less than 0.25l, preferably smaller than 0.1l, most preferably less than 0.025l.

The calibration and/or verification tool may be lightweight, and may have a weight of less than 250g, preferably lighter than 100g, most preferably less than 25g.

Within this application "disposable" means, that the calibration and/or verification tool can be disposed in common household garbage, as it meets relevant waste requirements. In particular, "disposable" means that the device does not comprise electronic components (besides possible passive detection means) or any material, which is toxic or explosive.

The interface may comprise a sealing element. The sealing element provides for a connection between the compartment and the contact face in a sufficiently sealing manner, preferably in a gas tight manner. The sealing element can be an O-ring made from an elastomer, such as Ethylene Propylene Diene Rubber (EPDR), Polyacrylate Rubber (ACM), Chloroprene Rubber (CR), Fluorocarbon Rubber (FKM), and Silicone Rubber (VMQ).

The calibration and/or verification tool may comprise a removeable packaging sealing element. Alternatively, or additionally, the calibration and/or verification tool may comprise a removeable gas impermeable packaging.

Within this application "gas impermeable" means sufficiently gas tight for the intended purpose. In particular, this means that ambient CO2 shout be sufficiently kept out for the duration of the intended shelf life (typically 24 months).

In view of the relatively low partial pressure, the packaging may comprise MAP (modified atmosphere packaging) materials, used e.g. for food packaging.
For calibrating and/or verification tools that are based on the use of gas with a known CO2 concentration or CO2 absorbing material, packaging is a key factor for their shelf lifespan. A gas impermeable packaging material can extend the lifespan of the device. The lifespan of the device can also be extended by filling the package with a defined concentration of nitrogen or of any other calibration gas. This is important for the shipping and storage of the calibrating and/or verification tool.

The calibration and/or verification tool may comprise a humidity absorbing material for drying the reference environment, in particular for drying a reference gas provided in the compartment.

The calibration and/or verification tool may comprise means for providing a well-defined gas concentration in the compartment which is adapted for providing a carbon dioxide (CO2) concentration of less than 300ppm, preferably less than 100ppm, most preferably less than 50ppm, within the compartment.

In this case the calibration and/or verification tool is suitable for calibrating and/or verifying a system for measuring transcutaneous carbon dioxide (CO2) concentrations.

When connected to the system for measuring CO2 concentrations, the system should provide a value for the low CO2 concentration, which corresponds to the low CO2 concentration in the compartment. Otherwise, the system has to be adjusted.

This may be an automated process. It is not necessary to display the detected value to the user.

In this case the reference environment is essentially free of CO2 or at least there is a carbon dioxide concentration which is lower than the typical measurement accuracy of the system for measuring CO2 concentrations.

The calibration and/or verification tool may comprise means for providing a well-defined gas concentration which comprise or are formed by a carbon dioxide (CO2) absorbing material.

The CO2-absorbing material can be any chemical substance that is able to react with CO2 in a binding reaction. The technical term often used is that of "CO2 scrubber". It is preferable to use soda lime, which is a mixture based on Sodium Hydroxide (NaOH) and Calcium Oxide (CaO) or Calcium Hydroxide (Ca(OH)2). Other CO2 scrubbing materials can be for example Potassium Hydroxide (KOH), Lithium Hydroxide (LiOH) and Lithium Peroxide (Li2O2).

The absorbing material can be provided as a powder, as granulate or as fibres.

The granulate may comprise spherical granules, hemispheres, bullet shapes, broken fragments, or cylinders. The granulate particles may have pores and a large surface.

Most preferably, the absorbing material is provided as concave hemispheres since they provide a good gas diffusion while providing high surface area. Furthermore, they are easy to handle with minimal dust.

The CO2 absorbing material provides for removing essentially all CO2 from the compartment.

The calibration and/or verification tool may comprise a removable packaging sealing element for sealing the CO2 absorbing material with respect to the environment before use.

For example, a gas tight film, such as an aluminium foil, may be attached to a rim of the compartment which has to be pulled off before the contact face is connected to the interface.

The gas tight film may be attached to cover also the interface, such that fastening elements are protected before use.

Alternatively, before use the calibration and/or verification tool may be stored in a gas impermeable packaging.

After removal of the packaging sealing element, the compartment fills up with environmental air. After connection with the contact interface of the sensor, it takes some time until all carbon dioxide is removed from the compartment. Typically, the user may have to wait not more than three minutes, until the intended well-defined gas concentration is achieved.

Preferably, once the packaging sealing element or the package is removed, the calibration and/or verification tool should be used within a day, preferably within some minutes.

The absorbing material may be arranged in the compartment.
A separation element, such as a filter, a mesh or a sieve element, for example a perforated tube, may be arranged between the absorbing material and the interface, in particular between the absorbing material and the reference environment.

The filter, a mesh or a sieve element may provide for holding the absorbing material within the compartment and behind the separation element, in particular before and after use, when the calibration and/or verification tool is not connected to the sensor, and also may prevent contact between the absorbing material and the contact face during use. In particular, free flowing absorbing material is prevented from leaving the compartment. Particularly, in the case the absorbing material is provided as a thin powder, the mesh or filter must prevent it from getting in touch with the contact face of the sensor as it may affect the performance of the measurement and/or compromise the biocompatibility of the contact face.

The compartment may host a desiccant, which may absorb humidity and may dry the reference gas.

Water (H2O) is necessary for most CO2-absorbing materials to absorb carbon dioxide. However, too much water will reduce the efficiency of the CO2-absorption as the water may solidify the absorbing material. In some cases, water is a by-product of the reaction between the CO2 and CO2-absorbing material, which can increase the humidity inside the compartment. High humidity may influence CO2 measurements and affect the performance of the sensor.

In that case, a small amount of humidity absorbent may be added to the compartment before the start of the calibration/verification. The humidity absorbent can for example be a material based on silicon dioxide such as silica gel.

The compartment may comprise a housing with two parts, wherein the first part hosts a humidity absorbing material and the second part hosts the CO2 absorbing material.

Each part may be provided with an independent sealed packing, as the humidity absorbing material and the CO2 absorbing material should not be stored together to avoid drying of the absorber.

Additionally, both parts should not be exposed to ambient air, as ambient CO2 or water vapor may saturate the absorber or the desiccant, respectively.

The parts have to be combined and the respective sealings need to be broken for operation.

The compartment may be made of a material and with a thickness which are able to prevent the aging of the CO2-absorbing material. The material can be a polymer with a low CO2 diffusion coefficient such as polyethylene terephthalate (PET), polyimide (PI), polyoxymethylene (POM) or polyamide (PA).

The thickness of the compartment walls may be in a range from 1mm to 4mm.

The calibration and/or verification tool may comprise a pressurizing unit for moving a gas, in particular air or nitrogen, through the absorbing material.

The pressurizing unit may be arranged such that the gas may be pumped into and/or through the compartment.

The compartment may be flushed with the gas when the pressurizing unit is used.

The pressurizing unit may be a simple manual pump.

The pressurizing unit may comprise a syringe having a plunger. Absorbing material may be arranged within the syringe between the tip of the syringe and the part of the cylinder wherein the plunger moves for scrubbing the CO2. The compartment may be formed between the tip and the absorbing material.

The cylinder of the syringe may be prefilled with air or nitrogen, which is held between the plunger and the absorbing material before use.

Preferably, the calibration and/or verification tool comprises a thin long tube and/or a one-way valve for releasing gas from the compartment into the outer environment. A flow restrictor may be arranged in the long thin tube. The long thin tube for guiding gas out of the compartment may comprise a one-way valve at the distal end.

Within this application a "long thin" tube is tube which is longer, preferably at least ten times longer, more preferably at least 100 times longer, than the open diameter of the tube.

The plunger may be pressed to force air or nitrogen through the absorbing material, which removes CO2. Gas essentially free of CO2 may arrive in the compartment. Due to the flow, air may escape from the compartment through the one-way valve or long thin tube, preferably with a flow restrictor at the end, such that the compartment finally is filled with a low CO2 concentrated gas.

The pressurizing unit may be a manual pump for pumping air, preferably in a closed loop.

The calibration and/or verification tool may comprise a circular pipe, wherein a part of the circular pipe hosts the absorbing material and a part of the circular pipe forms the compartment and comprises an interface. The manual pump may be arranged as part of the circular pipe.

The calibration and/or verification tool comprises two one-way valves arranged, for example in the pipe, upstream and downstream the manual pump.

The CO2 absorbing material may be packed along the pipe and the manual pump moves the air in the pipe in a closed loop. Air may be forced to pass through the CO2 absorbing material multiple times in order to eliminate any CO2 traces. The compartment may be a part of the pipe, located between the manual pump and the absorbing material.

The means for providing a well-defined gas concentration may alternatively comprise a reservoir containing a reference gas with a CO2 concentration of less than 300ppm. The compartment may comprise a one-way valve or a long thin tube between the compartment and the outer environment. A pressure relief valve may be arranged between the reservoir and the compartment. The reservoir may be connected or connectable to a pressurizing unit for pushing the reference gas through the pressure relief valve.

The reservoir may comprise a housing with a material and a thickness which are able to maintain zero or very low CO2 concentration inside the reservoir. The material can be metal.

Alternatively, the material of the housing of the reservoir can be almost any material with no specific requirements for gas permeability in combination a with gas-tight packaging.

The calibration and/or verification tool may comprise a syringe and the pressurizing unit is formed by the plunger of the syringe. The interface may be arranged at the tip of the syringe and the compartment may be formed between the tip and the pressure relief valve.

The part of the cylinder of the syringe below the pressure relieve valve and above the plunger may form the reservoir and may be filled with reference gas with a CO2 concentration of less than 300ppm, for example with nitrogen.

After the sensor has been attached to the interface, the plunger may be pressed forward in direction towards the tip. The reference gas is pressed through the pressure relief valve and replaces the air escaping from the compartment through the one-way valve which also prevents environmental air from coming inside during calibration.

When the plunger is completely pressed in, the compartment is filled with the reference gas of low or no CO2 concentration.

As an alternative, the calibration and/or verification tool may comprise a vacuum device for creating a vacuum within the compartment.

The vacuum device may comprise a plunger, which may provide for a sudden increase of the volume in the cylinder of a syringe and thus for a vacuum.

The calibration and/or verification tool may comprise a syringe, wherein the interface may be arranged at the tip of the syringe. Before use the plunger may be arranged close to the interface.

The plunger may be made of or may have a contact surface comprising a deformable material, which allows providing a seal, such as a rubber. When the plunger is moved backward, a low pressure or vacuum is created in the space between plunger and a tip which forms the compartment.

The plunger may be tensioned by a spring mechanism, which may be released to allow a rapid backward movement of the plunger.

Within this context "vacuum" means that the compartment comprises a CO2 concentration which is below the measurement accuracy of the system.

The plunger may comprise a one-way valve, allowing gas to escape from the compartment between the tip and the plunger. The plunger may be moved forward and backward several times to pump the gas out of the compartment.

The means for providing a well-defined gas concentration in the compartment may comprise at least two substances, which substances when reacting with each other generate a gas with a well-defined gas concentration.

The calibration and/or verification tool may comprise a one-way valve or a long thin tube between the compartment and the outer environment, such that the generated gas urges the any gas present within the compartment out of the compartment through the one-way valve or the long thin tube.

As an example, nitrogen may be produced by the reaction of the two substances.

A possible reaction is described by the following example:
Sodium nitrite-solution (NaNO2) may react with a solid sulfamic acid (HSO3NH2) to a solid NaHSO4, liquid water H2O and gaseous nitrogen (N2).

At the end of the reaction and after environmental air has been expelled from the compartment, the contact face may be exposed to a pure nitrogen atmosphere.

The substances may be placed in the compartment separated by a removeable or repositionable dividing element, such that when the dividing element has been removed or replaced, the substances may be allowed to contact each other and to react.

The calibration and/or verification tool may comprise a syringe, wherein the interface is arranged at the tip of cylinder. The substances and a dividing element may be placed in the cylinder, a first substance may be solid and may be placed on a first side of the dividing element close to the tip and a second substance may be liquid and may be placed on the other side of the dividing element. Before use the dividing element may seal the upper from the lower part of the compartment within the cylinder.

Forward movement of the plunger may press the liquid substance placed in the lower part of the cylinder towards the dividing element which may be repositioned or may be destroyed in order to open the seal. The dividing element may for example be a movable block which may be pushed to a region of the cylinder having a larger diameter, such that the liquid substance may enter the upper part of the cylinder and the reaction may start.

Alternatively, at least one substance may be arranged in the compartment and the dividing element may be a membrane in a housing of the compartment. A second substance may be arranged in a container which may be at least partially sealingly insertable into the compartment through the membrane, and which may be adapted to be opened during or after insertion to release the second substance into the compartment.

The calibration and/or verification tool may comprise means for providing a well-defined gas concentration in the compartment which is adapted for providing a CO2 concentration of a well-defined value higher than 300ppm in the compartment or in a reservoir being sealingly connected or connectable with the compartment.

Preferably the CO2 concentration is in the range of 3 to 130, more preferably in a range between 6 to 10%.

The calibration tool may comprise a reservoir with a reference gas, in this case a gas with a well-defined CO2 concentration of higher than 300ppm.

The reservoir may comprise a housing which preferably is metallic, for example a pressurized steel canister, to prevent any diffusion of the pressurized calibration gas.

The compartment may comprise a one-way valve and/or a long thin tube between the compartment and the outer environment. Preferably a flow restrictor is arranged at the distal end of the long thin tube.

The reservoir may be attached or attachable to the compartment, such that after attachment a flow restrictor is arranged between the reservoir and the compartment and such that the reference gas is able to enter the compartment through the flow restrictor and to establish a reference environment in the compartment.

Preferably the reference gas is under pressure. As soon as the flow restrictor is opened, for example by attaching the reservoir to the compartment, the reference gas may enter the compartments and replace air, which escapes into the environment through the one-way valve or through the long thin tube, preferably with a flow restrictor at the end. The compartment may be flushed by the reference gas.

Finally, the compartment is filled with reference gas.

Alternatively, the calibration tool may comprise a reservoir containing a reference gas with a CO2 concentration of higher than 300ppm. The compartment comprises a one-way valve or a thing long tube arranged between the compartment and the outer environment. A pressure relief valve, for example a one-way valve is arranged between the reservoir and the compartment.
The reservoir may be connected to a pressurizing unit for pushing the reference gas through the pressure relief valve.

An assembly for calibrating and/or verification of a system for measuring gas concentrations comprises a system for measuring gas concentrations and a calibrating and/or verification tool as described above.

In particular, the system is a system for measuring of gases that have diffused transcutaneously, in particular CO2.

The system has one or more sensors for detection of transcutaneous gas, in particular for detection of CO2. The sensor comprises a contact face which is directable towards a measuring site on the skin of a human or animal subject. The system has a monitor.

Within this application a monitor is not just the screen or display, but a medical device to monitor patient's vital signs.

The monitor may comprise or may be connectable to a display.

The monitor does not necessarily have to comprise a calibration function.

However, a system having a monitor comprising a calibration function may still be used with the calibrating and/or verification tool. So, the calibration function of the monitor does not have to be maintained.

The calibrating and/or verification tool has a compartment and an interface for connecting the sensor, such that the contact face is exposed to a reference environment in the compartment.

The monitor or sensor may comprise a reader for reading the detection means mounted to the calibrating and/or verification tool. The monitor or sensor may comprise a control unit which is adapted to receive data from the detection means, to start and/or to perform a calibrating and/or verification process. This may be a mostly automated process, controlled by software.

The control unit may be adapted to compare the measured concentration detected by the monitor with the gas concentration provided by the calibration and/or verification tool and to initiate a recalibration of the sensor, if the difference between the measured concentration and the gas concentration is larger than a predetermined threshold.

A method for calibrating and/or verifying an assembly for measuring gas concentrations having a sensor with a contact face which is directable towards a measuring site and having a monitor comprises the step of using a calibration and/or verification tool as described above.

The calibration and/or verification tool has a compartment and an interface for connecting the sensor, such that the contact face is exposed to a reference environment in the compartment.
The method further comprises the steps of coupling the contact face to the interface of the calibration tool.

Subsequently it is checked if the measured concentration detected by the monitor corresponds to the gas concentration provided by the reference environment in the compartment of the calibration and/or verification tool.

In particular, if needed, the sensor is calibrated by adjusting the measured concentration detected by the monitor on basis of the gas concentration provided by the verification and/or calibration tool.

A recalibration may be necessary if the concentration detected by the monitor differs from gas concentration provided by the calibration and/or verification tool by more than a predetermined concentration range. The range may depend on the gas concentration provided by the verification and/or calibration tool.

A final result of the calibration and/or verification process and/or a status along the process may be displayed by the system for measuring CO2 concentrations, such that a user knows if the process has started, is ongoing or has been finished.

The invention is further explained with reference to preferred embodiments and the following drawings which show:
- Fig. 1: a first example of a calibration and/or verification tool in a sectional view;
- Fig. 2: the first example of the calibration and/or verification tool in a perspective view shown without the sealing element (6);
- Fig. 3: a second example of a calibration and/or verification tool;
- Fig. 4: a third example of a calibration and/or verification tool;
- Fig. 5: a fourth example of a calibration and/or verification tool;
- Fig. 6a and Fig. 6b: two embodiments of a fifth example of a calibration and/or verification tool;
- Fig. 7: a sixth example of a calibration and/or verification tool;
- Fig. 8: a seventh example of a calibration and/or verification tool and
- Fig. 9: an assembly comprising a calibration and/or verification tool and a system.

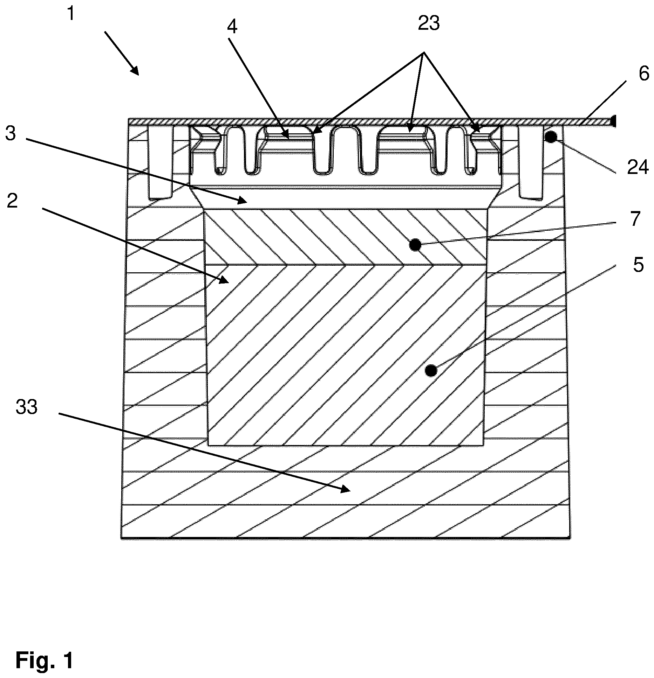

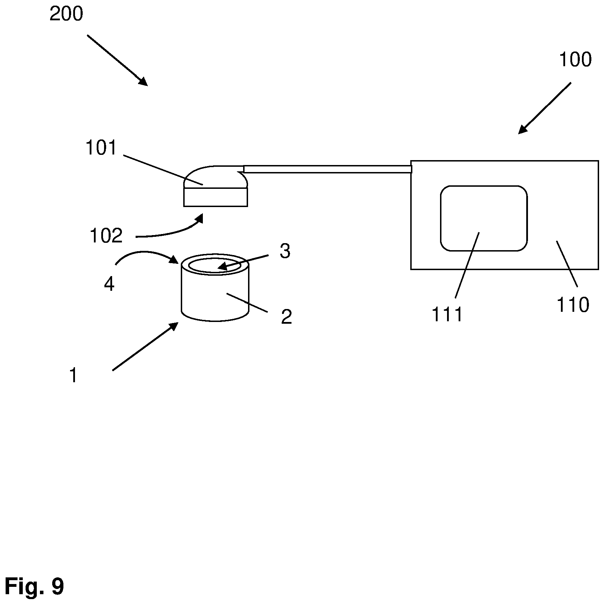

Figure 1 shows a first example of a calibration and/or verification tool 1 in a sectional view. The calibration and/or verification tool 1 is a tool for calibration and/or verification a system 100 (see figure 9) for measuring gas concentrations, with a sensor 101 (see figure 9) for detection of transcutaneous gas, comprising a contact face 102 (see figure 9) which is directable towards a measuring site on the skin of a human or animal subject.

The calibration and/or verification tool 1 is a mobile, stand-alone device and comprises a compartment 2 for encompassing a reference environment 3.

The calibration and/or verification tool 1 comprises an interface 4 for connecting the sensor 101 (see figure 9), such that the contact face 102 is exposed to the reference environment 3 in the compartment 2. In this example the interface 4 comprises clamping elements 23 for fastening the sensor 101.

The calibration and/or verification tool 1 comprises means for providing a well-defined gas concentration in the compartment, in this case a carbon dioxide absorbing material 5, arranged in the compartment 2.

The carbon dioxide absorbing material 5 provides a carbon dioxide concentration of less than 300ppm within the compartment 2.

A filter 7 is arranged between the absorbing material 5 and the interface 4, to prevent the absorbing material, typically a granular substance from leaving the compartment or polluting the interface 4 and the sensor 101.

The calibration and/or verification tool 1 comprises a packaging sealing element 6 which is mounted to a rim 24 of the compartment and which protects the compartment 2 including the filter 7 and the interface 4 before use.

The interface 4 could carry a sealing element (not shown in the figure) such as an O-ring, but it is not necessary as an increase in the amount of the CO2-absorbing material can compensate for potential leaks. The same holds for the second, third and fourth example as shown below.

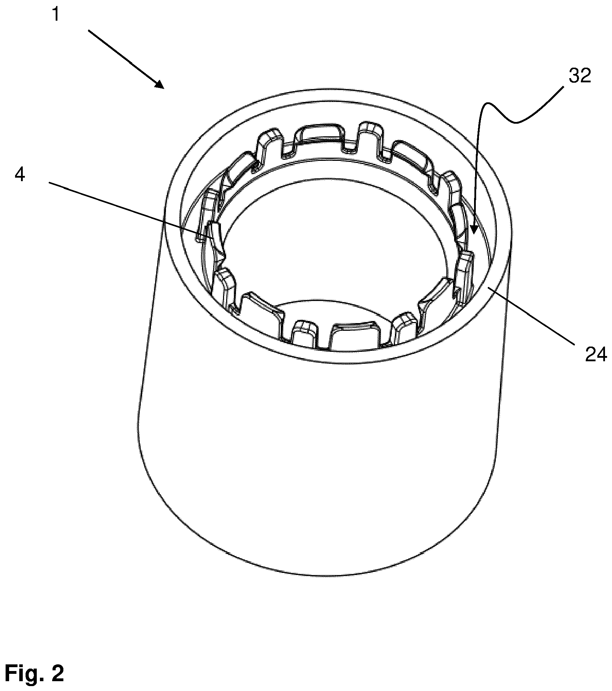

Figure 2 shows the first example of the calibration and/or verification tool 1 in a perspective view without the sealing element 6 (see figure 1), the filter 7 (see figure 1) and the absorbing material 5 (see figure 1).

An adhesive ring 32 comprising an antenna and/or a chip may be arranged between the interface 4 and the rim 24 as a detection means. Alternatively, an antenna and/or a chip may be arranged in the bottom part 33 (see figure 1) of the calibration and/or verification tool 1.

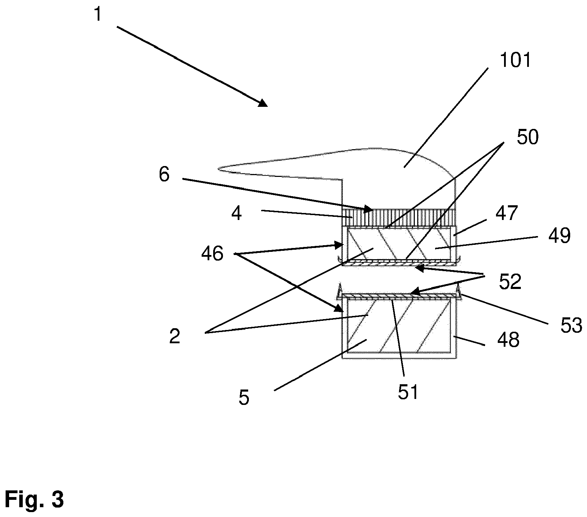

Figure 3 shows a second example of a calibration and/or verification tool 1. The second example is similar to the first example, wherein the means for providing a well-defined gas concentration in the compartment 2 of the calibration and/or verification tool 1 comprises a carbon dioxide absorbing material 5 arranged in the compartment 2.

The compartment 2 comprises a housing 46 with two parts 47, 48.

The first part 47 of the housing 46 comprises the interface 4 for attaching the sensor 101 and houses a humidity absorbing material 49. Filters 50 may hold the humidity absorbing material 49 at place.

The second part 48 of the housing 46 is filled with absorbing material 5, which is held in place by a filter 51.

Before use each part 47, 48 of the housing is separately closed by a gas tight film or foil 52, for example an aluminium foil.

Before attaching the sensor 101 to the interface 4, the sealing element 6 and the foils 52 shall be removed from the parts 47, 48 of the housing 46. The second part 48 can be attached to the first part 47 and is secured by connecting clips 53.

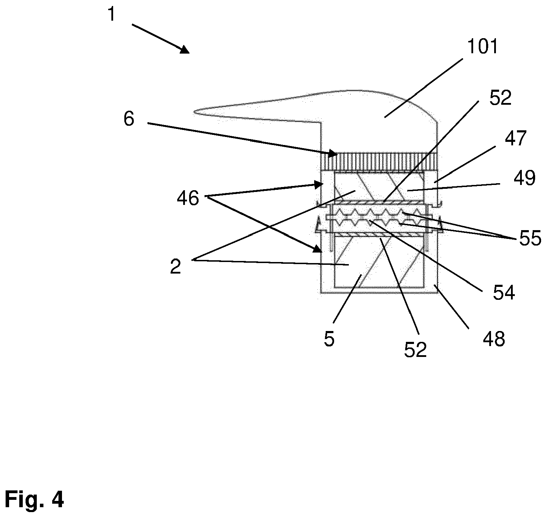

Figure 4 shows a third example of a calibration and/or verification tool 1 which is similar to the second example shown in figure 3.

The compartment 2 comprises a housing 46 with two parts 47, 48, wherein the first part 47 is filled with a humidity absorbing material 49 and the second part 48 is filled with CO2 absorbing material 5.

A perforated plate 54 is attached to the first part 47 of the housing and arranged such that after mounting the second part 48 to the first part 47 the perforated plate 54 is arranged between the first part and 47 and the second part 48 of the housing 46. The perforated plate 54 comprises pricking elements 55 on both sides.

Before use the user presses the second part 48 to the first part 47, then the pricking elements 55 break both gas tight foils or films 52, for example aluminium foils, which do not have to be pulled up before use. Then after removing sealing element 6 the sensor 101 can be attached to the interface 4.

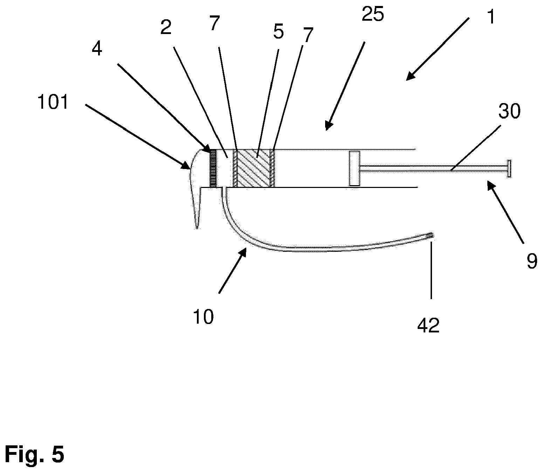

Figure 5 shows a fourth example of a calibration and/or verification tool 1.

The calibration and/or verification tool 1 comprises a pressurizing unit 9 for moving a gas, in particular air or nitrogen, through the absorbing material 5. The pressurizing unit 9 comprises a syringe 25 having a plunger 30.

Absorbing material 5 is placed in the syringe 25 and is separated and hold in place by filters 7. The interface 4 for connecting the sensor 101 is formed at the tip of the syringe 25.

The compartment 2 is formed between the interface 4 and the absorbing material 5.

When the plunger 30 of the syringe 25 is pushed inwardly, a gas, for example air or nitrogen, is pressed through the absorbing material 5 into the compartment 2 and CO2 is removed from the gas.

A one-way valve, in this case formed by a long thin tube 10 with an optional flow restrictor 42 at the end, arranged in the compartment allows gas to escape from the compartment 2 such that finally only CO2 free gas is in the compartment 2.

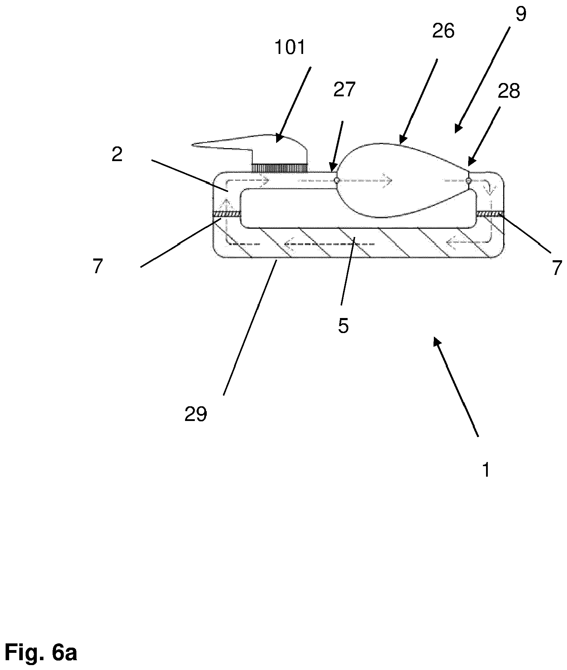

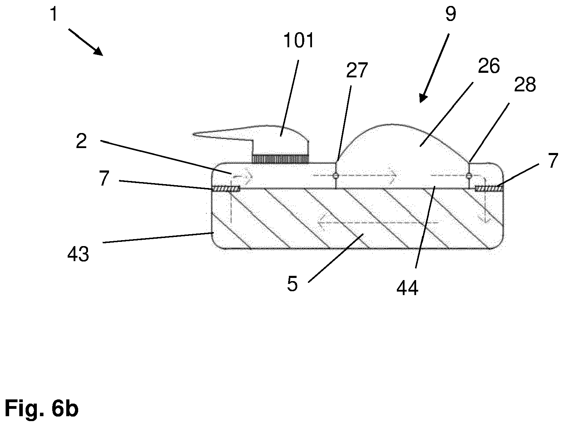

Figures 6a and 6b show two embodiments of a fifth example of a calibration and/or verification tool 1.

The calibration and/or verification tool 1 comprises a pressurizing unit 9 which is formed as a manual pump 26 for guiding air in a closed loop through an absorbing material 5.

A first one-way valve 27 is arranged upstream and a second one-way valve 28 is arranged downstream the manual pump 26, such that the gas may be only pumped in one direction.

The compartment 2 is arranged between the absorbing material and the manual pump 26, upstream of the one-way valve 27.

According to the first embodiment shown in figure 6a, the manual pump 26 is integrated in a circular pipe 29, which hosts the absorbing material 5. The absorbing material 5 is arranged in the circular pipe 29 and separated by filters 7.

According to the second embodiment shown in figure 6b, the manual pump 26, the compartment 2 and the absorbing material 5 are integrated in a box 43. The absorbing material 5 is separated by an inner wall 44 and by filters 7, which allow to pump gas in a circle.

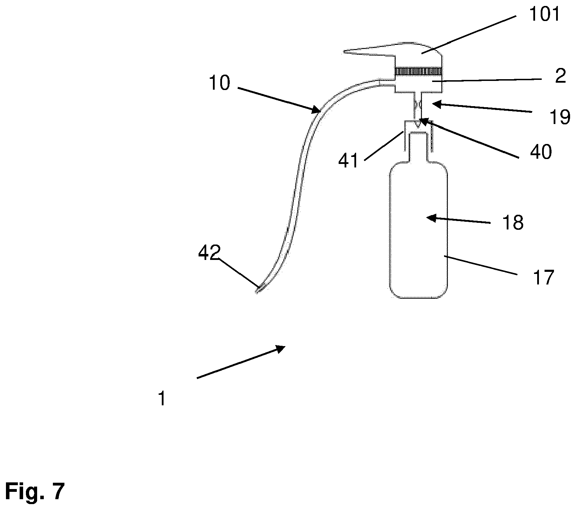

Figure 7 shows a sixth example of a calibration and/or verification tool.

In this example, the calibration tool 1 comprises a reservoir 17 containing a pressurized reference gas 18 with a well defined CO2 concentration, for example a concentration of 8% or of a nearly zero concentration, which may achieved for example by a nitrogen filling.

The reservoir 17 may be provided as a metal cartridge with a compressed calibrated reference gas 18.

The compartment 2 comprises a flow restrictor 19, an adaptor 41 and a pricking pin 40 that breaks the ceiling of the metal cartridge 17 when the reservoir 17 is attached to the compartment 2.

After attachment the flow restrictor 19 is arranged within the gas path of reservoir 17. The reference gas 18 is able to enter the compartment 2 through the flow restrictor 19.

The compartment 2 comprises a long thin tube 10 between the compartment 2 and the outer environment which prevents air from the outside entering the compartment 2 and allows gas to escape from the compartment 2. A flow restrictor 42 may additionally be arranged at the end of the long thin tube 10.

After a short time, the compartment 2 is completely filled with reference gas.

The interface 4 may comprise a sealing element (not shown in the figure) which is optional as the pressure in the compartment 22 will be higher than the environmental pressure so any leaks will have the direction to the environment.

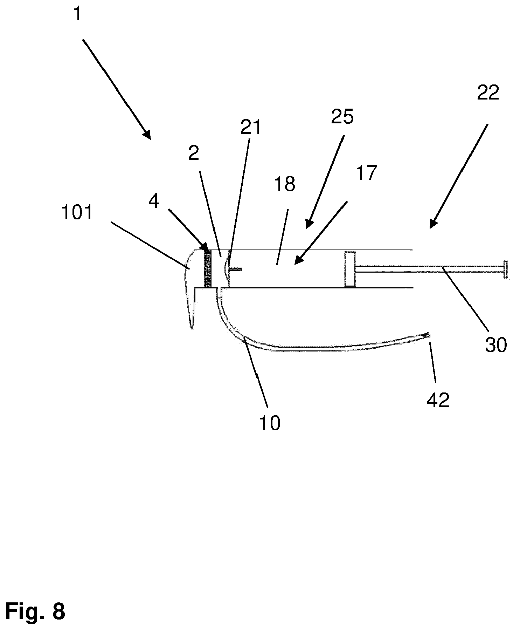

Figure 8 shows a seventh example of a calibration and/or verification tool 1 before use.

The calibration and/or verification tool 1 comprises a reservoir 17 containing a reference gas 18, for example a gas with a CO2 concentration smaller than 300ppm, preferably close to zero. In this example the compartment 2 and the reservoir 17 are arranged in a syringe.

A pressure relief valve 21 is arranged between the reservoir 17 and the compartment 2. The reservoir 17 is connected to a pressurizing unit 22, in this case a plunger 30 of the syringe 25, for pushing the reference gas 18 through the pressure relief valve 21 into the compartment 2.

The compartment 2 comprises a one-way valve, in this case a long thin tube 10 with an optional flow restrictor 42 at the end, which prevents environmental air from entering the compartment 2 and which allows gas to escape from the compartment 2. After the sensor 101 has been connected to the compartment 2, the plunger 30 can be pushed inwardly and press the reference gas into 18 the compartment 2, such that the compartment 2 is completely filled with reference gas 18 and the calibration and/or verification tool 1 can be used for verification and/or calibration.

The interface 4 preferably comprises a sealing element (not explicitly shown in the figure). The sealing element such as an O-ring is necessary because the CO₂-free gas in the reservoir 17 is not pressurized and a pressurized flow in the compartment 2 will be maintained only for a few seconds. The sealing element may guarantee a low CO₂ concentration in the compartment 2 after the end of the flow, throughout a calibration process.

Figure 9 shows an assembly 200 comprising a calibration and/or verification tool 1 and a system 100.

The system 100 is suitable for measuring gas concentrations, in particular for measuring of gases that have diffused transcutaneously, in particular CO2.

The system 100 comprises a sensor 101 for detection of transcutaneous gas, in particular for detection of CO2, with a contact face 102 which is directable towards a measuring site on the skin of a human or animal subject.

The system 100 comprises a monitor 110 with a display 111.

The sensor 101 can be attached to the calibration and/or verification tool 1 having an interface 4 for connecting the sensor 101, such that the contact face 102 is exposed to a reference environment 3 in the compartment 2 of the calibration and/or verification tool 1.

After the contact face 102 has been coupled to the interface 4 of the calibration tool 1, it may be automatically checked if the measured concentration, optionally displayed on the display 111 of the monitor 110, corresponds to the gas concentration provided by the reference environment 3 in the compartment 2.

If needed, the sensor 101 may be recalibrated by adjusting the measured concentration displayed on the display 111 on basis of the gas concentration provided by the verification and/or calibration tool 1.

## Claims

1. Calibration and/or verification tool (1) for calibrating and/or verifying a system (100) for measuring gas concentrations, with a sensor (101) for detection of transcutaneous gas, comprising a contact face (102) which is directable towards a measuring site on the skin of a human or animal subject,
the calibration and/or verification tool (1) comprising
- a compartment (2) for encompassing a reference environment (3),
- an interface (4) for connecting the sensor (101), such that the contact face (102) is exposed to the reference environment (3) in the compartment, and
- means (5; 18) for providing a well-defined gas concentration in the compartment,
wherein the calibration and/or verification tool (1) is a mobile stand-alone device and preferably a single-use and/or a disposable device.

2. Calibration and/or verification tool according to claim 1, wherein the calibration and/or verification tool comprises a detection means, which is adapted to be recognized and/or to be read-out by the system (100) for measuring gas concentrations.

3. Calibration and/or verification tool according to claim 1 or 2, wherein the calibration and/or verification tool (1) comprises a removeable packaging sealing element (6) and/or the calibration and/or verification tool (1) comprises a removeable gas impermeable packaging.

4. Calibration and/or verification tool according to one of the claims 1 to 3, wherein the calibration and/or verification tool comprises a humidity absorbing material (49) for drying the gas in the reference environment (3).

5. Calibration and/or verification tool according to one of the previous claims, wherein the means (5) for providing a well-defined gas concentration in the compartment (2) are adapted for providing a carbon dioxide concentration of less than 300ppm, preferably less than 100ppm, most preferably less than 50ppm, within the compartment (2).

6. Calibration and/or verification tool according to claim 5,
wherein the means (5) for providing a well-defined gas concentration comprise or are formed by a carbon dioxide absorbing material (5), in particular soda lime.

7. Calibration and/or verification tool according to claim 6,
wherein the absorbing material (5) is arranged in the compartment (2), and
preferably a separation element (7), in particular a filter (7), a mesh or a sieve element is arranged between the absorbing material (5) and the interface (4), in particular between the absorbing material (5) and the reference environment (3).

8. Calibration and/or verification tool according to claim 6 or 7, further comprising a pressurizing unit (9; 26) for moving a gas, in particular air or nitrogen, through the absorbing material (5).

9. Calibration and/or verification tool according to claim 8,
wherein the pressurizing unit (9) comprises a syringe (25) having a plunger (30),
and wherein the calibration and/or verification tool preferably comprises a thin long tube (10) and/or a one-way valve for releasing gas from the compartment (2) formed by a part of the syringe (25) into the outer environment.

10. Calibration and/or verification tool according to claim 8, wherein the pressurizing unit is a manual pump (26) and wherein the calibration and/or verification tool preferably comprises two one-way valves (27, 28) arranged upstream and downstream the manual pump (26).

11. Calibration and/or verification tool according to claim 5,
wherein
the calibration and/or verification tool comprises a reservoir (17) containing a reference gas (18) with a CO2 concentration smaller than 300ppm,
wherein the compartment (2) comprises a thin long tube (10), preferably with a flow restrictor (42), and/or a one-way valve between the compartment (2) and the outer environment, and wherein a pressure relief valve (21) is arranged between the reservoir (17) and the compartment (2) and
wherein the reservoir is connected to a pressurizing unit (22) for pushing the reference gas (18) through the pressure relief valve (21).

12. Calibration and/or verification tool according to one of the claims 1 to 4, wherein the means (18) for providing a well-defined gas concentration in the compartment are adapted for providing a CO2 concentration of a well-defined value higher than 300ppm, in particular in the range of 3 to 130, more preferably in the range of 6 to 10%, in the compartment or in a reservoir being sealingly connected or connectable with the compartment.

13. Calibration and/or verification tool according to claim 12,
wherein
the calibration tool (1) comprises a reservoir (17) containing a pressurized reference gas (18) with a CO2 concentration of higher than 300ppm,
the compartment comprises a long thin tube (10), preferably with a flow restrictor (42), and/or a one-way valve between the compartment and the outer environment, and
wherein the reservoir (17) comprises a flow restrictor (19) and the reservoir (17) is attached or attachable to the compartment (2), such that the flow restrictor (19) is arranged between the reservoir (17) and the compartment (2) and such that the reference gas is able to enter the compartment (2) through the flow restrictor (19) and to establish a reference environment (3) in the compartment (2).

14. Calibration and/or verification tool according to claim 12,
wherein
the calibration tool (1) comprises a reservoir (17) containing a reference gas (18) with a CO2 concentration of lower than 300ppm,
the compartment comprises a long thin tube (10), preferably with a flow restrictor (42), and/or a one-way valve between the compartment and the outer environment,
a pressure relief valve (21) is arranged between the reservoir (17) and the compartment (2), and
the reservoir (17) is connected to a pressurizing unit (22) for pushing the reference gas (18) through the pressure relief valve (21).

15. Assembly (200) for calibrating and/or verification of a system for measuring gas concentrations (100),
comprising a system (100) for measuring gas concentrations, in particular a system for measuring of gases that have diffused transcutaneously, in particular CO2, the system (100) having a sensor (101) for detection of transcutaneous gas, in particular for detection of CO2, the sensor (101) comprising a contact face (102) which is directable towards a measuring site on the skin of a human or animal subject, and the system (100) having a monitor (110),
the assembly for calibration (200) further comprising a calibration and/or verification tool (1) according to one of claims 1-14, having a compartment (2) and an interface (4) for connecting the sensor (101), such that the contact face (102) is exposed to a reference environment (3) in the compartment, the calibration and/or verification tool (1) preferably comprising a detection means, which may be recognized and/or read out by the system for measuring gas concentrations (100).

16. Method for calibrating and/or verifying an assembly (200) for measuring gas concentrations having a sensor (101) with a contact face (102) which is directable towards a measuring site and having a monitor (110),
using a calibration and/or verification tool (1) according to one of claims 1-14, having a compartment (2) and an interface (4) for connecting the sensor (101), such that the contact face (102) is exposed to a reference environment (3) in the compartment,
comprising the following steps,
- coupling the contact face (102) to the interface (4) of the calibration tool (1),
- optionally, recognizing and/or reading out a detection means of the calibration and/or verification tool (1),
- checking if the measured concentration detected by the monitor (110) corresponds to the gas concentration provided by the reference environment in the compartment (2) of the calibration and/or verification tool (1),
- preferably, if needed, recalibrating sensor by the adjusting the measured concentration detected by the monitor (110) on basis of the gas concentration provided by the verification and/or calibration tool.
